Europäisches Patentamt

European Patent Office

Office européen des brevets

(11) Publication number: **0 355 218 B1**

# EUROPEAN PATENT SPECIFICATION

(45) Date of publication of patent specification: **01.09.93**   (51) Int. Cl.⁵: **C07C  1/04**

(21) Application number: **88307572.3**

(22) Date of filing: **16.08.88**

The file contains technical information submitted after the application was filed and not included in this specification

(54) **Process for catalytic hydrocarbon synthesis from CO and H2 over metallic cobalt.**

(43) Date of publication of application:
**28.02.90 Bulletin  90/09**

(45) Publication of the grant of the patent:
**01.09.93 Bulletin  93/35**

(84) Designated Contracting States:
**DE FR GB NL**

(56) References cited:
**EP-A- 0 109 702**
**DE-C- 487 379**
**DE-C- 716 853**
**NL-C- 77 441**
**US-A- 2 497 964**

(73) Proprietor: **EXXON RESEARCH AND ENGI-
NEERING COMPANY
P.O.Box 390, 180 Park Avenue
Florham Park, New Jersey 07932(US)**

(72) Inventor: **Kim, Chang Jung
RR. No. 1 Rattlesnake Bridge Road
Bedminster New Jersey 07921(US)**

(74) Representative: **Somers, Harold Arnold et al
ESSO Engineering (Europe) Ltd. Patents &
Licences Mailpoint 72 Esso House Ermyn
Way
Leatherhead, Surrey KT22 8XE (GB)**

EP 0 355 218 B1

## Description

This invention relates to the Fischer-Tropsh synthesis of hydrocarbons, more especially $C_5 +$ hydrocarbons.

Background of the Invention

The production of hydrocarbons from mixtures of $H_2$ and CO via the Fischer-Tropsch process is well known to those skilled in the art. As opposed to the well-known "methanization" process which produces methane as synthetic natural gas from mixtures of $H_2$ and CO, the Fischer-Tropsch process is more generally aimed at producing higher value products such as chemical feedstocks and liquid fuels. Thus, high methane make is undesirable in Fischer-Tropsch synthesis processes because it is a relatively low value product which is formed at the expense of more desirable products. It is also uneconomical to try to convert the so-formed methane back into a CO and $H_2$ mixture and recycle it back into the reactor.

Methane make in Fischer-Tropsch reactions is often expressed by a term known as methane selectivity. Methane selectivity can be defined by either of two methods. They are, (a) mole % $CH_4$ produced based on the amount of CO consumed or (b) weight % of $CH_4$ produced based on total hydrocarbon products formed.

Many different catalysts and processes have been disclosed for Fischer-Tropsch synthesis, some of which have extremely high methane make. Thus, U.S. Patent 4,077,995 discloses synthesis of $C_1$-$C_4$ aliphatic hydrocarbons over a catalyst comprising a sulfided mixture of CoO, $Al_2O_3$ and ZnO while U.S. Patent 4,039,302 discloses $C_1$-$C_3$ hydrocarbon production using a mixture of the oxides of Co, Al, Zn and Mo. U.S. Patent 4,151,190 discloses producing $C_2$-$C_4$ hydrocarbons from mixtures of CO and $H_2$ using a supported catalyst comprising a metal oxide or sulfide of Mo, W. Re, Ru, Ni or Pt plus an alkali or alkaline earth metal, with Mo-K on carbon being preferred. U.S. Patent Nos. 4,243,553 and 4,243,554 disclose $MoS_2$ as a Fischer-Tropsch catalyst. Many other catalysts are known to be useful for Fischer-Tropsch synthesis employing metals such as iron, copper, titania, etc. These are known to those skilled in the art.

The type of catalyst used and process conditions employed have an important bearing on $CH_4$ selectivity. For example, nickel gives a high $CH_4$ selectivity and is used mainly as a methanization catalyst. Methane selectivity usually increases with increasing temperature, decreasing pressure and increasing the $H_2$/CO ratio of the feed. Accordingly, process conditions are selected so as to minimize $CH_4$ selectivity while maintaining a relatively high reaction rate as is well known to those skilled in the art.

It is known that $CH_4$ selectivity is influenced by the choice of promoter and support, such as alkali metal promoters reducing $CH_4$ selectivities of iron catalysts. It is also known in the art that noble metals such as ruthenium supported on inorganic refractory oxide supports exhibit superior hydrocarbon synthesis characteristics with relatively low methane production. Thus, U.S. Patent No. 4,088,671 suggests minimizing methane production by using a small amount of Ru on a cobalt catalyst. Examples of supported ruthenium catalysts suitable for hydrocarbon synthesis via Fischer-Tropsch reactions are disclosed in U.S. Patent Nos. 4,042,614 and 4,171,320. It is also known that the type of support used also influences methane production. In the case of supported ruthenium catalysts, the use of a titania or titania containing support will result in lower methane production than, for example a silica, alumina or manganese oxide support.

EP-A-109,702 relates to a Fischer-Tropsch hydrocarbon synthesis process employing, as a catalyst, cobalt supported on silica promoted with zirconium, titanium or chromium wherein the cobalt is deposited on the silica carrier by a kneading process. In this process, 10 to 40 volume percent steam is added to the feed, based on the $H_2$/CO/$H_2$O mixture. The amount of cobalt present in the catalyst is 10-40 pbw based on 100 pbw of silica. In the Examples, very little enhancement in either CO conversion or $C_3^+$ selectivity is shown as a result of the water addition to the feed. U.K. patent application 2146350A relates to a Fischer-Tropsch hydrocarbon synthesis process employing a similar catalyst, followed by a hydrocracking process wherein the Fischer-Tropsch products are hydrocracked over a noble metal catalyst. The same type of catalyst is also used in the Fischer-Tropsch processes described in U.K. patent application nos. 2,149,812A and 2,149,813A.

U.S. Patent 3,927,999 relates to a process for producing a methane rich fuel gas containing 70 to 98 mole % methane by contacting a mixture of $H_2$, CO and $H_2$O with a suitable catalyst at elevated temperature. This invention is based on the discovery that the methane content of the product gas from the methanator is maximized by adjusting the mole % $H_2$O in the synthesis gas feed to a critical value in the range of 1 to 3, while maintaining the $H_2$/CO mole ratio of the synthesis gas feed to a critical value in the range of about 1 to 1.15. The feed gas may also contain methane. Group VIII transition metals such as iron, nickel and cobalt are suggested as suitable catalysts, but only nickel on alumina is actually used. Example 1 shows the production of a fuel gas containing 96.4 mole % $CH_4$ from a feed gas containing 48.1 mole % $CH_4$ over a catalyst containing nickel, thoria, magnesia and kieselguhr.

U.S. Patent 2,479,439 relates to a process for alternately increasing the activity of an alkali metal promoted, powdered iron catalyst or decreasing the coke buildup thereon, or accomplishing both simultaneously by passing an aqueous solution of alkali metal salt, such as KF, into the hydrocarbon synthesis reaction zone to contact the iron catalyst. The presence of the water is said to remove carbon from the iron catalyst. U.S. Patent 2,539,847 relates to a Fischer-Tropsch hydrocarbon synthesis process employing a catalyst consisting of thoria promoted cobalt supported on bentonite. U.S. Patent 4,568,663 relates to a Fischer-Tropsch hydrocarbon synthesis process employing a rhenium cobalt on titania catalyst.

The Fischer-Tropsch (FT) process, the process to which this invention relates, must also be distinguished from the well known Kolbel-Engelhardt (KE) process (described, for example, in Canadian Patent No. 530,932). The FT process may be described by equation (1), the KE process by equation (2).

$$2nH_2 \ + \ nCO \text{ -}(CH_2)_n\text{- } + \ nH_2O \qquad (1)$$

$$nH_2O \ + \ 3nCO \text{ -}(CH_2)_n\text{- } + \ 2nCO_2 \qquad (2)$$

The two processes may appear similar in that they yield similar products and can employ Group VIII metals as catalysts. The reactions are not similar because their reaction paths differ in the formation of hydrocarbons and because FT yields $H_2O$ as a product while KE consumes $H_2O$ as a reactant. The reactions can be distinguished in that in FT there is no net consumption of $H_2O$, whereas in KE there is net consumption of $H_2O$. Even if hydrogen is added to the reaction mixture for KE, water will be consumed.

There exists a need in the art for Fischer-Tropsch processes useful for the conversion of mixtures of CO and hydrogen to $C_5+$ hydrocarbons at high CO conversion levels, and high hydrocarbon yields, with relatively low methane make.

EP-A-339,923 of earlier priority date describes a Fischer-Tropsch hydrocarbon synthesis with added water, utilising a catalyst of promoted or unpromoted cobalt supported (with dispersion) on titania.

The invention provides a process for the Fischer-Tropsch synthesis of $C_5+$ hydrocarbons in which hydrogen and carbon monoxide are reacted in a reaction zone at elevated temperature and pressure in the presence of a catalyst, characterised in that

i. from 1 to 70 vol.% water (or a suitable precursor) is added to the hydrogen and carbon monoxide, based on the total volume,

ii. the catalyst is arranged in a fixed bed, and

iii. the catalyst comprises cobalt in reduced form:

(a) in the form of powder or granules, optionally alone or mixed with a solid diluent, or

(b) surface-supported without dispersion on a support of metal or inorganic oxide.

The water (or precursor) may be added to the feed prior to entering the reaction zone or added directly to the the reaction zone.

By cofeeding $H_2O$ into the reaction zone, along with the $H_2$ and CO, it has been found that $C_5+$ hydrocarbon production is increased, CO conversion is increased and $CH_4$ production is decreased. By cobalt is meant cobalt in the reduced, metallic form, preferably a high surface area cobalt like cobalt black.

The $H_2O$ that is added to the reaction zone may be in the form of steam or moisture or a suitable $H_2O$ precursor, such as $C_1$-$C_6$ alcohols, for forming $H_2O$ in-situ in the reaction zone. It is essential to the understanding of the process of this invention that the $H_2O$ introduced into the reaction zone is external $H_2O$ and not that $H_2O$ which is formed in-situ in the reaction zone as a consequence of the Fischer-Tropsch hydrocarbon synthesis reaction from the $H_2$ and CO. It has also been found that the process of this invention improves with increasing pressure in the reaction zone and with decreasing CO conversion.

In a preferred embodiment of the invention, the synthesis gas feed, carbon monoxide and hydrogen is substantially free of and preferably completely (except for trace amounts) free of methane or other light hydrocarbons. The hydrocarbon free synthesis gas feed is easily accomplished in a once-through system where there is no recycle of unconverted hydrogen and carbon monoxide (along with undesirable light hydrocarbons) directly to the hydrocarbon synthesis reaction zone. Preferably, the synthesis gas feed contains only $H_2$, CO and water and is substantially free of light hydrocarbons, e.g., methane. Allowable limits on light hydrocarbons in the synthesis gas feed are no more than about 1 vol.% and preferably less than about 0.5 vol.%.

## DETAILED DESCRIPTION

The process of the present invention resides in adding, to the Fischer-Tropsch reaction zone, $H_2O$ or a suitable $H_2O$ precursor such as an alcohol. The amount of $H_2O$ added to the reaction zone will range from about 1-70 volume % of the total feed mixture of $H_2O$, CO and $H_2$ and, preferably, from about 5-30 volume %. As the extent of CO conversion increases, the amount of water produced in-situ in the reaction zone increases and, concomitantly the beneficial effect of introducing additional water into the reaction zone to increase CO conversion, reduce $CH_4$ selectivity and increase $C_5$ + hydrocarbons selectivity decreases. Thus, in some cases, it may be advantageous to practice the process of this invention at CO conversion levels below about 60 percent. That is, below about 60% per pass, reaction zone or stage. As the pressure in the reaction zone increases, the beneficial effect of the process of this invention of adding water to the reaction zone increases with respect to increasing CO conversion activity, decreased $CH_4$ selectivity and increased $C_5$ + hydrocarbon selectivity. At relatively low pressures in the reaction zone (i.e.; less than about 1 atmosphere), little effect will be seen in increased conversion activity, etc. by adding $H_2O$ to the reaction zone. Thus, the process of this invention will be operated at a pressure above atmospheric. In general the pressure will range from above atmospheric to 5065 kPa (50 atmospheres), and more preferably 506.5-4052 kPa 5-40 atmospheres).

In the process of this invention, the addition of $H_2O$ enhances the formation of $C_5$ + hydrocarbons and reduces $CH_4$ make in a uni-directional or linear fashion. It has also been found that the rate of the Fischer-Tropsch reaction employing the process of this invention increases with increasing partial pressure of $H_2O$ at any fixed total pressure in the reaction zone, up to a point, after which point the rate slowly goes down with continually increasing $H_2O$ partial pressure.

In general, the Fischer-Tropsch hydrocarbon synthesis reaction process of this invention is carried out at a $H_2$:CO mole ratio of greater than 0.5, and preferably the $H_2$:CO mole ratio ranges from 0.5 to 6, more preferably from 0.5 to 3, at gas hourly space velocities ranging from 100 V/Hr/V to 5000 V/Hr/V, preferably from 300 V/Hr/V to 1500 V/Hr/V, at temperatures ranging from 150°C to 300°C, preferably from 180°C to 240°C, and pressures above 101.3 kPa (1 atm.), ranging e.g. up to 5065 kPa (50 atm.), preferably from 506.5 kPa (5 atm.) to 4052 kPa (40 atm.) and more preferably 506.5-3039 kPa (5-30 atmospheres).

As previously stated, the hydrocarbon synthesis process of this invention employs a catalyst comprising cobalt in its reduced, i.e. elemental or metallic form, preferably a high surface area cobalt like cobalt black. The cobalt may be in the form of a finely divided powder, or granules which may be mixed with a suitable diluent to aid in heat transfer and removal from the reaction zone. The cobalt may be supported on a suitable support material, such as cobalt plated on carrier metal. However, this is not meant to include cobalt dispersed on inorganic refractory oxide supports. The cobalt in bulk form, or plated, explosion-coated, etc. may also be in the form of various high surface area shapes such as spirals, metal wool, honeycomb configurations, etc., the choice being left to the practitioner.

The invention will be more readily understood by reference to the examples below.

## EXAMPLES

### Example 1

A bulk cobalt catalyst, designated as a cobalt black catalyst, was prepared by a conventional method known in the art, i.e., adding a stoichiometric amount of ammonium carbonate (as an aqueous solution) to an aqueous solution of cobalt nitrate, filtering the precipitate, washing the filtered solid with deionized water, drying at 120°C, and calcining at 500°C in air for 5 hours. The resulting material showed an X-ray diffraction pattern of $Co_3O_4$ which, when reduced at 450°C in a $H_2$ stream, produced a cobalt black catalyst having a surface area of 7.7 m²/g.

The performance test of this catalyst was carried out by charging an intimate mixture of the $Co_3O_4$ powder (6.8 grams) and a diluent (quartz powder, 177$\mu$m-105$\mu$m (80-140 mesh), 18 grams) into a down-flow fixed bed reactor made of 9.5mm (3/8") OD stainless steel tube with a concentric 3.2mm (1/8") OD thermocouple well. When reduced, 6.8 grams of $Co_3O_4$ will yield 5.0 grams of cobalt black. The use of diluent and an aluminum block jacket fitted tightly around the reactor minimized uneven temperature profile along the bed. The $Co_3O_4$ was then re-reduced in-situ in the reactor in a flowing $H_2$ stream (200 cc/m, atmospheric pressure) overnight at 450°C, cooled to 175°C and then pressurized to 2026 kPa (20 atm.) using a pre-mixed gas composed of 63.9% $H_2$, 32.1% CO and 4.0% $N_2$ with different proportions of steam. At pressure and at a preset flow rate, the temperature was raised to 200°C over a period of one hour and then data acquisition was initiated. The rate of CO conversion and the rates of various hydrocarbon products formation were monitored using two on-line GC's. The data taken after 70 hour on-stream-time are shown in Table 1.

<div align="center">

TABLE 1

EFFECTS OF EXTERNAL $H_2O$
ADDITION ON ACTIVITY
SELECTIVITIES OF CO BLACK

</div>

Temperature = 200°C
Feed Gas Composition = 63.1% $H_2$/33.0% CO/3.9% $N_2$
SV = 3600 Scm$^3$/g hr (calcined for $H_2$ and CO)*1

| | Run 1 | Run 2 | Run 3 | Run 4 |
|---|---|---|---|---|
| % $H_2O$ Added*2 | 0 | 12.5 | 27.7 | 50.0 |
| Total Pressure, kPa (atm.) | 2097(20.7) | 2188(21.6) | 2522(24.9) | 2847(28.6) |
| P$H_2$+pco, kPa (atm.) | 2026(20.0) | 1894(18.7) | 1915(18.9) | 1884(18.6) |
| P$H_2O$, kPa (atm.) | 0 | 233(2.3) | 527(5.2) | 942(9.3) |
| % CO Conv. | 12.1 | 27.3 | 28.7 | 18.8 |
| $CH_4$ Select*3 | 10.5 | 7.1 | 5.8 | 4.0 |
| $CO_2$ Select*3 | 0.27 | 0.21 | 0.34 | |

Hydrocarbon Product Distribution, wt.%

| | Run 1 | Run 2 | Run 3 | Run 4 |
|---|---|---|---|---|
| $CH_4$ | 11.5 | 8.1 | 6.7 | 4.6 |
| $C_2$-$C_4$ | 15.6 | 8.7 | 7.8 | 8.6 |
| $C_5$-$C_9$ | 17.4 | 9.5 | 9.0 | 9.7 |
| $C_{10}$+ | 55.5 | 73.7 | 76.5 | 77.1 |
| $C_{10}$-$C_{20}$ | 21.7 | 17.0 | | |
| $C_{21}$-$C_{30}$ | 21.7 | 13.8 | | |
| $C_{31}$-$C_{40}$ | 6.9 | 11.1 | | |
| $C_{41}$-$C_{50}$ | 4.7 | 8.1 | | |
| $C_{51}$+ | 11.8 | 23.7 | | |

*1   cc of $H_2$ and CO measured at atmospheric pressure, 22°C per gram of catalyst per hour.

*2   Moles of $H_2O$ added per 100 moles of CO and $H_2$

*3   Moles of $CH_4$ or $CO_2$ per 100 moles of CO converted

Comparison of the data with and without the external $H_2O$ addition clearly establishes the advantages of $H_2O$ addition; (1) the CO conversion increases markedly (up to 2.5 fold increase), (2) the methane make is greatly reduced and, (3) the desired heavy hydrocarbon product yields, which may be gauged by the $C_{10}$ + selectivity, increase dramatically.

Thus, this example clearly demonstrates the benefits of conducting a Fischer-Tropsch hydrocarbon synthesis reaction with external $H_2O$ addition in the presence of a catalyst comprising metal cobalt.

## Example 2

Another batch of a bulk cobalt catalyst was prepared using highly purified $Co(NO_3)_2 \cdot 6H_2O$ (99.99% pure Puratronic grade) according to a procedure similar to that described in Example 1. The evaluation procedure was also identical to that used in Example 1. After an extended reduction in a flowing $H_2$/He mixture at 500°C, the material was passivated and found to have about 1.5 $m^2$ surface area per gram. This catalyst (5 grams) and 10 grams of quartz powder were mixed and charged into the reactor and pretreated as described in Example 1. At 201.5°C and 2097 kPa (20.7 atm.), a gas mixture composed of 63.1% $H_2$, 33.0% CO and 3.9% $N_2$ was passed at a rate of 105 $Scm^3$/m. After one day on stream the data in Table 2 were obtained.

## TABLE 2

### EFFECTS OF ADDED $H_2O$ ON THE ACTIVITY AND SELECTIVITIES OF CO HYDROGENATION OVER A HIGH-PURITY BULK CO CATALYST

Temperature = 202°C
Feed Gas Composition = 63.1% $H_2$/33% CO/3.9% $N_2$
Pressure = 20 atm. ($2026$ $kPa$)
SV = 1200 $Scm^3$/g hr

|  | Run 5 | Run 6 |
|---|---|---|
| % $H_2O$ Added | 0 | 21 |
| % CO Conversion | 7.2 | 15.6 |
| $CH_4$ Selectivity | 8.2 | 4.2 |
| 1-olefin/paraffin Ratio | | |
| $C_2$ | 0.13 | 0.36 |
| $C_3$ | 2.47 | 3.68 |
| $C_4$ | 1.87 | 2.52 |
| Hydrocarbon Product Distribution, wt.% | | |
| $CH_4$ | 9.4 | 4.8 |
| $C_2-C_4$ | 21.1 | 20.4 |
| $C_5-C_9$ | 11.0 | 11.6 |
| $C_{10}+$ | 58.5 | 63.2 |

## Claims

1. A process for the Fischer-Tropsch synthesis of $C_5+$ hydrocarbons in which hydrogen and carbon monoxide are reacted in a reaction zone at elevated temperature and pressure in the presence of a catalyst, characterised in that
   i. from 1 to 70 vol.% water (or a suitable precursor) is added to the hydrogen and carbon monoxide, based on the total volume,
   ii. the catalyst is arranged in a fixed bed, and
   iii. the catalyst comprises cobalt in reduced form:
      (a) in the form of powder or granules, optionally alone or mixed with a solid diluent, or
      (b) surface-supported without dispersion on a support of metal or inorganic oxide.

**2.** The process of claim 1 wherein the water (or precursor) is added to the feed prior to entering the reaction zone.

**3.** The process of claim 1 wherein the water (or precursor) is added directly to the the reaction zone.

**4.** The process of any preceding claim wherein the pressure in the reaction zone is from 506.5 to 4052 kPa (5 to 40 atm.)

**5.** The process of any preceding claim wherein the temperature in the reaction zone is from 150° to 300°C.

**Patentansprüche**

**1.** Verfahren zur Fischer-Tropsch-Synthese von $C_5+$-Kohlenwasserstoffen, bei dem Wasserstoff und Kohlenmonoxid in einer Reaktionszone bei erhöhter Temperatur und erhöhtem Druck in Gegenwart eines Katalysators umgesetzt werden, dadurch gekennzeichnet, daß
    i. dem Wasserstoff und Kohlenmonoxid 1 bis 70 Vol.% Wasser (oder ein geeigneter Vorläufer) zugesetzt werden, bezogen auf das Gesamtvolumen,
    ii. der Katalysator in einem Festbett angeordnet ist, und
    iii. der Katalysator Kobalt in reduzierter Form umfaßt:
        (a) in Form von Pulver oder Körnern, wahlweise allein oder mit einem festen Verdünnungsmittel gemischt, oder
        (b) ohne Dispergierung auf der Oberfläche eines Trägers aus Metall oder anorganischem Oxid aufgetragen.

**2.** Verfahren nach Anspruch 1, bei dem das Wasser (oder der Vorläufer) dem Einsatzmaterial vor Eintreten in die Reaktionszone zugesetzt wird.

**3.** Verfahren nach Anspruch 1, bei dem das Wasser (oder der Vorläufer) direkt in die Reaktionszone gegeben wird.

**4.** Verfahren nach einem der vorhergehenden Ansprüche, bei dem der Druck in der Reaktionszone 506,6 bis 4 052 kPa (5 bis 40 atm) beträgt.

**5.** Verfahren nach einem der vorhergehenden Ansprüche, bei dem die Temperatur in der Reaktionszone 150°C bis 300°C beträgt.

**Revendications**

**1.** Procédé de synthèse Fisher-Tropsch d'hydrocarbures en $C_5+$, dans lequel l'hydrogène et l' oxyde de carbone sont mis en réaction dans une zone réactionnelle à température et pression élevées en présence d'un catalyseur, caractérisé en ce que :
    i. on ajoute 1 à 70 % en volume d'eau (ou un précurseur approprié) à l'hydrogène et à l' oxyde de carbone par rapport au volume total,
    ii. le catalyseur est disposé dans un lit fixe, et
    iii. le catalyseur comprend du cobalt sous forme réduite :
        (a) sous la forme de poudre ou de granules, éventuellement seul ou en mélange avec un diluant solide, ou
        (b) le cobalt est supporté en surface sans dispersion sur un support d'oxyde de métal ou d'oxyde minéral.

**2.** Procédé selon la revendication 1, dans lequel l'eau (ou le précurseur) est ajoutée à la charge d'alimentation avant que celle-ci ne pénètre dans la zone réactionnelle.

**3.** Procédé selon la revendication 1, dans lequel l'eau (ou le précurseur) est ajoutée directement dans la zone réactionnelle.

4. Procédé selon l'une quelconque des revendications précédentes, dans lequel la pression dans la zone réactionnelle est de 506,5 à 4.052 kPa (5 à 40 atm.).

5. Procédé selon l'une quelconque des revendications précédentes, dans lequel la température de la zone réactionnelle est de 150°C à 300°C.